# EUROPÄISCHE PATENTSCHRIFT

(11) **EP 3 178 464 B1**
(45) Veröffentlichungstag und Bekanntmachung des Hinweises auf die Patenterteilung: **21.08.2024**
(21) Anmeldenummer: 16202698.3
(22) Anmeldetag: 07.12.2016
(51) Int. Cl.: C04B 41/00, C04B 41/87, C04B 41/50, A61C 13/00, A61C 13/08, A61C 13/083, A61K 6/78, A61K 6/802, A61K 6/818, A61K 6/822, A61K 6/824, C04B 111/00, C04B 111/82

(54) **VERFAHREN ZUR HERSTELLUNG EINES IMPLANTATROHLINGS**
METHOD FOR PRODUCING AN IMPLANT BLANK
PROCÉDÉ DE FABRICATION D'UNE ÉBAUCHE D'IMPLANT

(30) Priorität: 07.12.2015 DE 102015121246; 12.05.2016 EP 16169366; 12.05.2016 EP 16169371
(43) Veröffentlichungstag der Anmeldung: 14.06.2017
(73) Patentinhaber: WDT-Wolz-Dental-Technik GmbH, 55566 Bad Sobernheim (DE)
(72) Erfinder: Wolz, Stefan, 55566 Bad Sobernheim (DE)
(74) Vertreter: Götz, Georg Alois

(56) Entgegenhaltungen:
- CN-A- 1 080 627
- US-A1- 2009 118 114
- DATABASE CAPLUS, [online] 3 October 1994 (1994-10-03), XIE JIANLIN ET AL: "BIOLOGICAL ACTIVE GRADIENT CERAMIC MATERIALS", XP002510701, retrieved from CAPLUS Database accession no. 1994-562585 162585
- XIANGMING LI ET AL: "Fabrication and properties of porous Si3N4-SiO2 ceramics with dense surface and gradient pore distribution", CERAMICS INTERNATIONAL., vol. 40, no. 3, 1 April 2014 (2014-04-01), NL, pages 5079 - 5084, XP055335087, ISSN: 0272-8842, DOI: 10.1016/j.ceramint.2013.09.068

## Beschreibung

Die Erfindung betrifft ein Verfahren zur Herstellung eines Implantatrohlings, insbesondere eines Dentalimplantatrohlings aus einem Ausgangskörper. Unter einem Implantatrohling wird insbesondere das noch nicht dicht- bzw. endgesinterte Implantat verstanden. Der Implantatrohling umfasst mindestens einen ersten Bereich, der ein Oberflächenbereich ist und einen zweiten Bereich, der ein Kernbereich ist, wobei sich der Oberflächenbereich von mindestens einer ersten Oberfläche ausgehend in Richtung des Kernbereichs erstreckt. Vorzugsweise umfasst der Oberflächenbereich die erste Oberfläche des Implantatrohlings sowie ein darunter angeordnetes, oberflächennahes Volumen und geht, insbesondere nahtlos in den Kernbereich über, wobei der Kernbereich im Inneren des Implantatrohlings angeordnet ist. Der Oberflächenbereich weist mindestens ein bioaktives Oberflächenmaterial auf, und der Kernbereich mindestens ein mechanisch belastbares Trägermaterial.

In der Medizintechnik ist eine Vielzahl von Implantaten, die aus Implantatrohlingen hergestellt werden bekannt. Diese sind dazu gedacht in den menschlichen Körper eingepflanzt zu werden und dort körpereigene Funktionen zu übernehmen. Die Implantate können aus verschiedenen, keramischen und/oder metallischen, biokompatiblen Materialien hergestellt sein. Biokompatible Materialien (z. B. Aluminiumoxid, Zirkoniumoxid, Implantate aus Kunststoff, Titan, Niob und Tantal sowie ihre Legierungen) sind Materialien, die körperfremd sind, mit dem Empfängergewebe harmonieren und dabei keine inakzeptablen Reaktionen, wie beispielsweise Abstoßungsreaktionen auslösen. Sogenannte permanente Implantate, welche zumindest einen längeren Zeitraum oder auch permanent im Körper verbleiben sollen, weisen zumeist eine bioaktive Oberfläche bzw. Außenfläche auf, die ein bioaktives Material umfasst. Bioaktive Materialien (z. B. Calziumphosphatkeramiken wie Hydroxylapatit, Trikalziumphosphat, Glaskeramiken) lösen im Organismus spezifische, biologische Reaktionen aus, wodurch eine chemische Bindung zwischen Implantat und umliegendem Gewebe, insbesondere Knochen entsteht. Es handelt sich um körpereigene Materialien, die mit umliegenden Körperstrukturen in Wechselwirkung treten und auf diese Weise in den Körper des Patienten einheilen. Ferner unterscheidet man zwischen knochenresorbierenden und nicht knochenresorbierenden Materialien. Knochenresorbierende Materialien enthalten Calcium und/oder Magnesium, welches vom Körper zur Neubildung von Knochenstruktur herangezogen wird. Der Kernbereich eines Implantats umfasst ein mechanisch belastbares, vorzugsweise biokompatibles Material (z. B. Zirkonoxid-Keramiken oder Titan), welches zur Aufnahme von Kräften und/oder Momenten, die an dieser Stelle normalerweise auf die zu ersetzende Körperstruktur wirken, geeignet ist.

Unter Dentalimplantaten versteht man im Allgemeinen Implantate, die im Bereich der Zahnmedizin eingesetzt werden. Im Speziellen kann es sich hier um Zahnwurzelimplantate handeln, d.h. Implantate, die die Wurzel eines Zahns ersetzen und in den Kieferkamm des Patienten eingepflanzt werden. Ein weiteres Beispiel sind Knochenersatzmaterialien wie Kieferkammimplantate, welche Teile des oder sogar den vollständigen Kieferkamm des Patienten ersetzen. Zahnwurzelimplantate, also künstliche Zahnwurzeln bestehen aus einem Implantatkörper, der regelmäßig als Schraube mit Außengewinde ausgebildet ist und zur Einbringung bzw. zum Einschrauben in den Kieferknochen bzw. den Kieferkamm vorgesehen ist. An einen Übergangsabschnitt des Implantats, welcher im Bereich des Zahnfleischs des Patienten angeordnet ist, schließt sich ein sogenanntes Aufbauteil, auch Abutment genannt an. Das Abutment dient als Aufsatz für den eigentlichen Zahnersatz, zum Beispiel eine Zahnkrone, eine Brücke oder ähnliches. Das Abutment kann mit dem Implantatkörper sowie dem Übergangsabschnitt einteilig, d.h. einstückig oder auch zweiteilig, d.h. in den Übergangsabschnitt eingeschraubt, ausgeführt sein. Das Dentalimplantat ist vorzugsweise aus einem biokompatiblen, metallischen und/oder keramischen Material hergestellt. In der Dentaltechnik werden zumeist Titan oder Zirkonoxid verwendet, aber auch andere gewebeverträgliche, biokompatible Keramiken, (Dental-) Metalle und Metalllegierungen. Weitere Informationen zu (Dental-) Implantaten können den nachfolgenden Normen entnommen werden: ISO 14801 :2016 "Dentistry -- Implants -- Dynamic loading test for endosseous dental implants"; DIN EN ISO 5832-3:2015-06 - Entwurf "Chirurgische Implantate - Metallische Werkstoffe"; DIN EN ISO 5832-2:2012-08 "Chirurgische Implantate - Metallische Werkstoffe"; DIN EN ISO 16498:2013-12 "Zahnheilkunde - Mindestdatensatz bei der klinischen Anwendung von Dentalimplantaten"; DIN EN 1642:2012-06 "Zahnheilkunde - Medizinprodukte für die Zahnheilkunde - Dentalimplantate"; DIN EN ISO 13356:2016 "Chirurgische Implantate - Keramische Werkstoffe aus yttriumstabilisiertem tetragonalem Zirkoniumoxid (Y-TZP)"; ISO 13779-3: 2008 "Implants for surgery - Hydroxyapatite".

Um einen verbesserten Halt des Dentalimplantats innerhalb der Knochen- und/oder Gewebestruktur des Patienten zu erzielen wird der Implantatkörper eines Dentalimplantatrohlings, also der Teil, der in den Kieferkamm des Patienten einheilen soll, einer Oberflächenoptimierung zur Erhöhung der Biokompatibilität unterzogen. Hierzu wird generell zwischen einer chemisch/mechanischen Behandlung der Oberfläche sowie einer Beschichtung der Oberfläche des Implantatrohlings unterschieden.

Aus der DE 10 2007 007 865 A1 ist ein Verfahren zur Herstellung von Implantaten mit einer ultrahydrophilen Oberfläche bekannt. Oberflächen mit einer hohen Oberflächenenergie können eine starke Gewebe-Bioadhäsion aufweisen, die ein Einheilen des Implantats in den Organismus fördert. Oberflächen mit einer hohen Oberflächenenergie weisen zumeist niedrige Kontaktwinkel mit Wasser auf, die wiederum kennzeichnend für eine hohe Benetzbarkeit der Oberfläche sind. Zur Herstellung einer solchen ultrahydrophilen Oberfläche wird die Oberfläche eines Metallimplantats mit einem Oxidationsmittel zur Erzeugung einer Oxidschicht behandelt. Die Behandlung wird fortgesetzt bis sich eine Kontaktwinkel-Hysterese von weniger als 5° ergibt.

Die EP 2 046 235 B9 beschreibt ein Verätzungsverfahren zur Erzeugung einer Oberflächenstruktur an der Außenfläche eines Pfostenteils, d. h. des Implantatkörpers eines Dentalimplantats. Hierzu wird das Pfostenteil des Implantatrohlings, welcher aus einer Yttrium-stabilisierten Zirkonkeramik hergestellt ist, einem Ätzprozess unterzogen, wodurch selektiv einzelne Bestandteile aus der Oberfläche des Pfostenteils herausgelöst werden. Dort wo die Bestandteile herausgelöst wurden entstehen Poren im Nanometerbereich, die ein Einwachsen von umliegendem Knochen/Gewebe erleichtern.

Beispielsweise aus der WO 2014 101 997 A1 ist es bekannt, die Oberfläche eines Implantatrohlings mittels Sandstrahlen oder Schleifen anzurauen sowie zu verätzen, um die osteointegrativen Eigenschaften der Implantatoberfläche zu verbessern. Die Oberfläche des Rohlings wird hierbei mit einer Oberflächenstruktur versehen, die ein Einheilen des umliegenden Knochens in das Implantat erleichtert. Während des abschließenden End- bzw. Dichtsinterns, durch welches der Rohling die gewünschte mechanische Festigkeit erhält und zum fertigen Dentalimplantat wird, kommt es aufgrund der abrasiven Oberflächenbearbeitung jedoch häufig zur Bildung von Spannungen und Mikrorissen, welche das Dentalimplantat unbrauchbar machen und/oder zu einer hohen Reklamationsrate führen. Zur Lösung dieses Problems schlägt die WO 2014 101 997 A1 vor, die angeraute Oberfläche des Implantatrohlings aufwendig mit einer Beschichtung zu versehen. Hierzu wird zunächst ein Material auf die angeraute, biokompatible Oberfläche des Implantatrohlings aufgetragen. Der Rohling wird anschließend einer Wärmebehandlung unterzogen, wobei das Material in die bioaktive Oberfläche des Rohlings zur Verbesserung der mechanischen Festigkeit eindringt. Eine Veränderung der zuvor erzeugten Oberflächenrauigkeit und eine damit einhergehende Verschlechterung der physikalischen Eigenschaften sollte grds. vermieden werden.

Die DE 101 19 096 A1 offenbart eine biologisch funktionalisierte Beschichtung einer Implantatoberfläche. Die Beschichtung wird in einer Schichtdicke auf eine offenporige Substratoberfläche aufgebracht, welche die Offenporigkeit und somit die strukturellen Eigenschaften der Substratoberfläche nicht beeinträchtigt. Offenporige Substratoberflächen können beispielsweise durch bekannte Sinter-, Gieß- und Spritztechniken hergestellt werden. Bei metallischen Substraten können eine oder mehrere plasmagespritzte, korrosionschemisch optimierte Titanschichten auf dem Grundkörper aufgebracht werden. Die Substratoberfläche soll eine Porengröße zwischen 75 und 500 µm aufweisen, die Porosität soll zwischen 15 und 40 % liegen, um ein optimales Einheilen von Knochenstruktur zu ermöglichen.

Aus der WO 2015 168 332 A2 ist ein Verfahren zur Erzeugung von Oberflächenstrukturen auf einer Implantatoberfläche bekannt. Die Strukturierung verbessert die osteointegrativen Eigenschaften der Oberfläche wodurch ein verbesserter Knocheneinwuchs ermöglicht wird. Die Oberflächenstrukturierung wird bereits bei der Herstellung des Implantatrohlings erzeugt. Der Rohling kann beispielsweise durch Pulverkompaktierung, Schlickerguss, Injection Molding oder Spark Plasma Sintern hergestellt werden. Bevor der Rohling dicht- bzw. endgesintert wird, kann dessen strukturierte Oberfläche in eine wässrige, Ionen und/oder Partikel enthaltende Lösung getaucht werden. Zumindest ein Teil der Ionen und/oder Partikel treten in die strukturierte Oberfläche ein und werden durch den abschließenden Sintervorgang eingeschlossen.

Nachteilig an den zuvor beschriebenen Verfahren ist, dass durch die Behandlung eine Schwächung der mechanischen Eigenschaften der Implantatoberfläche oder des Implantatkörpers erfolgt. Dies kann insbesondere zu Mikrorissen führen, die während der weiteren Verarbeitung des Implantatrohlings oder auch während des späteren Gebrauchs des Implantats zu Beschädigungen führen.

Eine andere Möglichkeit zur Erzeugung einer biokompatiblen Oberfläche eines Implantats sind die sogenannten Beschichtungsverfahren. Hierzu wird die Oberfläche des Implantatkörpers beispielsweise mit biokompatiblen und/oder bioaktiven Verbundwerkstoffen zur Erzeugung einer hochporösen, ggf. bioaktiven Oberflächenbeschichtung versehen. Üblicherweise wird ein bioinerter, aber mechanisch stabiler Volumenwerkstoff als Hauptmasse des Implantates verwendet und mit einem Oberflächenwerkstoff beschichtet. Ein Nachteil der Beschichtungsmethode ist, dass zwischen der Implantatoberfläche und der Beschichtung ein mechanisch belastbarer Verbund erzielt werden muss, um ein Abplatzen der Beschichtung zu vermeiden. Im Falle des Abplatzens löst sich das Implantat aus dem umliegenden Gewebe und muss operativ entfernt werden.

Zu Beschichtung der Implantatoberfläche können insbesondere bioaktive Calciumphosphate wie beispielsweisen Hydroxylapatit (HAp) eingesetzt werden, welche dem mineralischen Bestandteil von biologischem Hartgewebe, wie Knochen, Dentin und Zahnschmelz ähneln. Die Beschichtung mit HAp erfolgt beispielsweise durch thermisches Plasmaspritzen. Hierbei wird pulverförmiges HAp mittels einer Plasmaflamme auf über 1650 °C erhitzt und mit hohem Druck auf die Implantatoberfläche aufgebracht. Die HAp-Partikel kühlen auf der Implantatoberfläche ab und erstarren zu einer festen, kristallinen Schicht. Alternative Möglichkeiten zur Beschichtung von Implantatoberflächen sind unter anderem Kathodenzerstäubung, Biomimetische Beschichtungsmethoden, elektrochemische, elektrolytische und elektrophoretische Beschichtungen sowie Sol-Gel-, Rotations- und Tauchziehverfahren (Friederike Kraas, "Über die Herstellung einer Oberflächenbeschichtung aus Hydroxylapatit mittels Sol-Gel-Synthese und Untersuchungen hinsichtlich ihrer in-vitro-Biokompatibilität" Dissertation, Kiel, 2014).

Aus der DE 10 2013 102 370 A1 ist beispielsweise ein Verfahren zur Herstellung eines funktionalisierten Implantats bekannt. Die Implantatoberfläche wird mit einem biokompatiblen Material beschichtet, welches zur Erzeugung einer hochporösen Oberfläche geeignet ist. Die Beschichtung erfolgt beispielsweise durch PVD-, CVD-Verfahren oder mittels elektrochemischer Prozesse. Aufgrund der porösen Beschichtung wird ein Einheilen der umliegenden Knochen- und/oder Gewebestruktur in das Implantat begünstigt. Um eine bessere Haftung zwischen der hochporösen Beschichtung und der Implantatoberfläche zu erreichen, kann diese vor Aufbringung der Beschichtung durch abrasive Oberflächenbehandlung angeraut werden. Das beschriebene Verfahren ist auch zur Herstellung von Dentalimplantaten geeignet.

Nachteilig an den beschriebenen Beschichtungsverfahren ist, dass zwischen der Beschichtung und der Implantatoberfläche kein ausreichend stabiler Verbund erzeugt werden kann, was zum Abplatzen der Beschichtung und somit zum Verlust des Implantats führen kann.

Aus der US 2009/118114 A ist eine bioaktive Glas/Keramik-Struktur zur Verwendung, unter anderem, als keramisches Zahnimplantat oder keramischer Zahnersatz bekannt. Die Struktur besteht aus einem dichten, inneren Keramikkern, einer an den Oberflächen angeordneten bioaktiven Glaskeramikschicht und einer darunter liegenden, graduellen Glaskeramikschicht. Das Glaskeramikmaterial wird mittels einer Infiltrationstechnik hergestellt. Hierzu werden vorgesinterte, noch poröse, zylindrische Zirkon-Keramikstangen in eine Ionen enthaltende Lösung getaucht, um das bioaktive Glaspulver an der Oberfläche der Zirkon-Keramikstangen niederzuschlagen, d. h. aus der Lösung zu fällen. Während des abschließenden Dichtsinterns infiltriert das aus der Lösung gefällte Glaspulver ausgehend von dessen Oberfläche, teilweise in die Poren des Keram ikkörpers.

Aufgabe der vorliegenden Erfindung ist es daher, ein verbessertes Herstellungsverfahren für einen Implantatrohling, insbesondere einen Dentalimplantatrohling, mit einer bioaktiven Oberfläche anzugeben. Es ist ferner Aufgabe der Erfindung einen verbesserten Implantatrohling, insbesondere einen Dentalimplantatrohling mit einer bioaktiven Oberfläche bereitzustellen.

Die Aufgabe wird durch ein Verfahren gemäß Anspruch 1 gelöst.

Vorteilhafte, optionale Ausbildungen und/oder Weiterbildungen ergeben sich ganz oder teilweise aus den abhängigen Ansprüchen.

Ein erfindungsgemäßes Verfahren der eingangs beschriebenen Art kennzeichnet sich dadurch, dass der Ausgangskörper eine Porosität zur Steuerung einer gezielten Verteilung des bioaktiven Oberflächenmaterials innerhalb des Ausgangskörpers aufweist. Als Ausgangskörper kann, insbesondere in der Dentalindustrie, ein poröser, herkömmlicher keramischer und/oder metallischer Rohling/Weißling/Grünling/Braunling verwendet werden, der zum Beispiel durch Pressen aus Keramikpulver oder Metallpulver, durch Schlickergießen aus keramischem oder metallischem Schlicker, mittels eines Keramik-3D-Druckers, durch Lasersintern (SLM), Spritzgießen oder ein anderes, geeignetes Verfahren hergestellt wurde. Bei Verwendung eines keramischen Ausgangskörpers kann dieser insbesondere mit Yttrium und/oder Calcium und/oder Cer stabilisiert sein, wobei der Anteil der Stabilisierungskomponente im Oberflächenbereich geringer ist als im Kernbereich, wodurch der Kernbereich eine erhöhte mechanische Stabilität aufweist.

Um eine gezielte Verteilung des bioaktiven Oberflächenmaterials innerhalb des Ausgangskörpers zu steuern, muss dieser eine Porosität aufweisen, d.h. der Ausgangskörper ist nicht dicht- bzw. endgesintert. Die Porosität des Ausgangskörpers liegt in einem Bereich zwischen 3 % und 90 %, vorzugsweise in einem Bereich zwischen 25 % und 30 %. Die Porosität ergibt sich aus dem Verhältnis der Rohdichte des Ausgangskörpers zu dessen Reindichte multipliziert mit 100 %.

In einem ersten Schritt, der ein Beladungsschritt ist, wird der Ausgangskörper mit einer oder mehreren Lösungen des bioaktiven Oberflächenmaterials beladen. Unter Beladung wird die Infiltration der Lösung, also des Lösungsmittels (Solvens) mit dem darin gelösten bioaktiven Oberflächenmaterial (Solvat) in das Innere, insbesondere auch in den Kernbereich des Ausgangskörpers verstanden.

In einem zweiten Schritt, der ein Verteilungssteuerungsschritt ist, wird die Verteilung des bioaktiven Oberflächenmaterials innerhalb des Ausgangskörpers derart gesteuert, dass die Lösung innerhalb des Oberflächenbereichs eine höhere Konzentration aufweist als innerhalb des Kernbereichs. Die Verteilung kann auch so gesteuert werden, dass die Konzentration im Kernbereich null ist. Eine höhere Konzentration der Lösung bedeutet insbesondere, dass mehr Lösung in den Poren des Ausgangskörpers vorhanden ist, aber auch eine höhere Konzentration des Solvats innerhalb des Solvens. Vorzugsweise wird ein gleichmäßiger Konzentrationsverlauf zwischen dem Oberflächen- und dem Kernbereich erzeugt. Der Verlauf kann zweidimensional oder dreidimensional ausgebildet sein

Nachdem der Ausgangskörper mit der Lösung des bioaktiven Oberflächenmaterials beladen wurde, kann dieses innerhalb des gesamten Volumens des Ausgangskörpers beliebig verteilt werden. Die Steuerung der Verteilung des bioaktiven Oberflächenmaterials erfolgt durch Regelung eines oder mehrerer Umgebungsparameter in einer abgeschlossenen Umgebung, insbesondere durch Regelung der Luftfeuchtigkeit und/oder des Drucks und/oder der Temperatur. Unter einer abgeschlossenen Umgebung wird eine Umgebung, die ein geschlossenes System bildet, z.B. ein Gefäß, Schrank, Raum oder ähnliches, welches gegen die Außenumgebung abgedichtet ist verstanden. Hierdurch kann eine gezielte Regelung der Umgebungsparameter erfolgen. Unter einer Regelung wird nicht ausschließlich eine quantitative Regelung, sondern auch eine Regelung bezüglich festgelegter, örtlicher Bereiche innerhalb der Umgebung verstanden, d. h. der Ausgangskörper kann bezüglich unterschiedlicher Oberflächen und/oder Oberflächenbereiche mit einem oder mehreren Umgebungsparametern beaufschlagt werden. Der eine oder die mehreren Beladungsschritte und der eine oder die mehreren Verteilungssteuerungsschritte können zeitlich aufeinanderfolgend oder (teilweise) gleichzeitig erfolgen.

Vorzugsweise erfolgt die Beladung des Ausgangskörpers mit der Lösung des mindestens einen, bioaktiven Oberflächenmaterials über eine zweite Oberfläche des Ausgangskörpers, die eine Beladungsfläche ist und von der ersten Oberfläche verschieden ist. D.h., die Lösung des bioaktiven Oberflächenmaterials wird während des Beladungsschritts über eine zweite Oberfläche, eine Beladungsfläche in das Innere, insbesondere den Kernbereich des Ausgangskörpers infiltriert. Während des Verteilungssteuerungsschritts wird die Lösung dem Oberflächenbereich, der sich von der ersten Oberfläche ausgehend in Richtung des Kernbereichs erstreckt und/oder der ersten Oberfläche selbst zugeführt.

Nach einer bevorzugten Verfahrensvariante wird die Beladungsfläche zur Beladung des Ausgangskörpers mit dem bioaktiven Oberflächenmaterial außerhalb der abgeschlossenen Umgebung angeordnet. Insbesondere befindet sich die erste Oberfläche des Ausgangskörpers innerhalb der abgeschlossenen Umgebung wohingegen die zweite Oberfläche, die Beladungsfläche außerhalb der abgeschlossenen Umgebung angeordnet ist. Die abgeschlossene Umgebung ist beispielsweise mittels einer geeigneten Dichtung, insbesondere einer Silikondichtung entlang des Ausgangskörpers gegen die Außenumgebung abgedichtet.

Nach einer optionalen Verfahrensvariante wird die Konzentration der Lösung während des Beladungsschritts und/oder während der Beladung des Ausgangskörpers konstant gehalten. D. h. das Verhältnis der Menge, insbesondere der Masse des bioaktiven Oberflächenmaterials zu der Gesamtlösungsmenge, insbesondere der Lösungsmasse wird konstant gehalten. Vorzugsweise erfolgt die Beladung mittels eines Beladungsreservoirs und einem Beladungskörper, wobei das Beladungsreservoir zuvor mit einem festgelegten Volumen der Lösung befüllt worden ist. Der Beladungskörper ist innerhalb des Beladungsreservoirs angeordnet und kann eine oder mehrere Beladungszonen umfassen. Jede Beladungszone kann entweder eine unterschiedliche Lösung aufweisen und/oder jeweils die gleiche Lösung mit unterschiedlicher Konzentration. Bei den unterschiedlichen Lösungen handelt es sich vorzugsweise um eine Lösung mit bioaktiven Oberflächenmaterial, eine Lösung mit chemischen Stoffen zur Beeinflussung physikalischer Eigenschaften, eine Lösung mit farbgebenden Komponenten oder auch um reines Lösungsmittel. Die Beladungszonen können für eine Parallelbeladung der Beladungsfläche nebeneinander angeordnet und beispielsweise durch Folien voneinander getrennt sein. Bei der Parallelbeladung werden unterschiedliche Lösungen und/oder dieselbe Lösung mit unterschiedlicher Konzentration zeitgleich, jedoch örtlich verschieden in den Ausgangskörper infiltriert. Für eine Reihenbeladung sind die Beladungszonen untereinander angeordnet und nicht voneinander getrennt. Bei der Reihenbeladung werden unterschiedliche Lösungen und/oder dieselbe Lösung mit unterschiedlicher Konzentration zeitlich aufeinanderfolgend, jedoch örtlich gleich in den Ausgangskörper infiltriert. Der Beladungskörper umfasst ein kapillardruckerhaltendes Material, insbesondere Mikrofasern. Die maximale Beladungsdauer des Ausgangskörpers ist abhängig vom Lösungsvolumen bzw. der Größe des Beladungsreservoirs. Um die Konzentration des Solvats innerhalb des Solvens, also den Anteil des bioaktiven Oberflächenmaterials innerhalb des Lösungsmittels konstant zu halten, ist das Beladungsreservoir gegenüber der Außenumgebung sowie gegenüber der abgeschlossenen Umgebung abgedichtet. Auf diese Weise wird eine Verdunstung des Lösungsmittels und somit eine Konzentrationserhöhung der Lösung verhindert. In einer alternativen Ausgestaltung kann das Beladungsreservoir einen Zufluss oder einen Zufluss und einen Abfluss aufweisen, um ein kontinuierliches Nachfüllen der Lösung aber auch eine Veränderung der Lösungskonzentration zu ermöglichen.

Die Verteilung des bioaktiven Oberflächenmaterials innerhalb des Ausgangskörpers wird durch eine Konvektionsströmung bewirkt. Hierbei wird eine Strömungsrichtung und/oder -geschwindigkeit der Lösung durch gezieltes Erzeugen von Umgebungsparametergradienten innerhalb der abgeschlossenen Umgebung gesteuert. Insbesondere werden Luftfeuchtigkeitsunterschiede und/oder Druckunterschiede und/oder Temperaturunterschiede bezüglich verschiedener Oberflächen und/oder bezüglich verschiedener Oberflächenbereiche des Ausgangskörpers eingestellt.

Gemäß einer vorteilhaften Ausführung des Verfahrens wird die Bewegungsgeschwindigkeit und/oder die Strömungsgeschwindigkeit der Lösung durch Erhöhen und/oder Verringern eines oder mehrerer der Umgebungsparametergradienten gesteuert. Beispielsweise kann ein erster Umgebungsdruck, der an einer Oberfläche des Ausgangskörpers anliegt erhöht werden, und ein zweiter Umgebungsdruck, der an einer anderen, gegenüberliegenden Oberfläche der porösen Keramik anliegt verringert oder konstant gehalten werden, wodurch der Betrag des Druckgradienten bezüglich der beiden Oberflächen erhöht wird. Dies führt wiederum zu einer Änderung, insbesondere einer Erhöhung der Bewegungsgeschwindigkeit und/oder der Strömungsgeschwindigkeit.

Gemäß einer ebenso vorteilhaften Ausführung des Verfahrens wird eine Bewegungsrichtung und/oder die Strömungsrichtung der Lösung durch Änderung der Richtung eines oder mehrerer der Umgebungsparametergradienten gesteuert. Beispielsweise kann der erste Umgebungsdruck an einer Oberfläche des Ausgangskörpers angelegt werden und der zweite Umgebungsdruck an einer anderen Oberfläche des Ausgangskörpers, wodurch die Bewegungs- und/oder eine Strömungsrichtung zwischen den Oberflächen des Ausgangskörpers verlaufend gesteuert wird. Durch eine Umkehr des Umgebungsparameter-Gradientenverlaufs (d. h. einer Änderung des Vorzeichens des Gradienten) kann die Bewegungsrichtung und/oder die Strömungsrichtung umgekehrt werden.

In einer optionalen Verfahrensvariante wird mindestens ein Teil der ersten Oberfläche des Ausgangskörpers während des Verteilungssteuerungsschritts gegenüber der abgeschlossenen Umgebung isoliert und/oder abgedichtet. Auf diese Weise können die Umgebungsparameter bezüglich festgelegter Oberflächen und/oder Oberflächenteilbereiche örtlich geregelt werden Dies dient der gezielten Steuerung der Verteilung, insbesondere der gezielten Steuerung der Bewegungsrichtung und/oder der Strömungsrichtung. Zusätzlich oder alternativ wird mindestens ein Teil der zweiten Oberfläche des Ausgangskörpers während des Beladungsschritts gegenüber der Außenumgebung und/oder gegenüber dem Beladungsreservoir isoliert und/oder abgedichtet. Die Isolierung und/oder Abdichtung kann während des Beladungsschritts zur örtlich gezielten Beladung des Ausgangskörpers verwendet werden. Vorteilhafterweise erfolgt eine Abdichtung und/oder Isolierung mittels einer Form, eines Gehäuses oder Ähnlichem und/oder einer Folie und/oder einer Beschichtung. Beispielsweise kann es sich bei der Form um eine Silikonform, bei der Folie um eine selbstklebende Folie und bei der Beschichtung um eine Silikon-, Latex- und/oder Wachsbeschichtung handeln.

Bei einer vorteilhaften Verfahrensvariante erfolgt die Steuerung der Konzentration und/oder des Konzentrationsverlaufs der Lösung innerhalb des Ausgangskörpers durch Trocknung und/oder Verdunstung. Konzentration der Lösung innerhalb des Ausgangskörpers bedeutet die Konzentration der Lösung, also die Menge des gelösten bioaktiven Oberflächenmaterials zur Gesamtlösungsmenge, aber auch die Lösungsmenge, die sich innerhalb des Ausgangskörpers, genauer in den Poren des Ausgangskörpers befindet. Insbesondere wird die erste Oberfläche oder zumindest ein Teil der ersten Oberfläche mit Wärme und/oder Luft bzw. einem Luftstrom beaufschlagt, um eine Verdunstung und/oder Verdampfung und/oder Trocknung der Lösung, insbesondere des Lösungsmittels zu erzielen. Auf diese Weise erhöht sich zum einen die Menge des Solvats, d.h. des bioaktiven Oberflächenmaterials gegenüber dem Solvens, d.h. gegenüber dem Lösungsmittel. Zum anderen strömt die Lösung aus dem Kernbereich des Ausgangskörpers in Richtung der ersten Oberfläche bzw. in Richtung der Teile der ersten Oberfläche, die zur Steuerung mit Wärme und/oder Luft beaufschlagt werden. Um eine Verdunstung des Lösungsmittels an der ersten Oberfläche des Ausgangskörpers zu ermöglichen kann zwischen der abgeschlossenen Umgebung und der Außenumgebung eine Verbindung, zum Beispiel in Form eines Ventils zur Abfuhr der Verdunstungs-, Verdampfungs-, und/oder Trocknungsfeuchtigkeit vorgesehen sein.

Vorzugsweise wird die Lösung des bioaktiven Oberflächenmaterials mittels des Beladungsschritts und/oder mittels des Verteilungssteurungsschritts derart in den Ausgangskörper infiltriert, dass ausgehend von der ersten Oberfläche eine Infiltrationstiefe des bioaktiven Oberflächenmaterials von mindestens 50 µm erzielt wird. Insbesondere wird das gesamte Volumen des Ausgangskörpers mit der Lösung beladen. Mittels des Verteilungsteuerungsschritts wird anschließend eine Strömung aus dem Kernbereich in Richtung des Oberflächenbereichs erzeugt, wodurch eine gezielte Infiltrationstiefe, ausgehend von der ersten Oberfläche, des bioaktiven Oberflächenmaterials gesteuert wird. Alternativ kann die Beladung des Ausgangskörpers über einzelne Teilbereiche der Beladungsfläche erfolgen, sodass beispielsweise ausschließlich der Oberflächenbereich mit der Lösung des bioaktiven Oberflächenmaterials beladen wird. Im Gegensatz zu den aus dem Stand der Technik bekannten Verfahren erfolgt die Verteilung des bioaktiven Oberflächenmaterials innerhalb des porösen Ausgangskörpers nicht durch Diffusion von dessen Oberfläche augehend in Richtung des Inneren, sondern andersherum aus dessen Innerem in Richtung der ersten Oberfläche. Durch dieses Verfahren lässt sich die Infiltrationstiefe gezielt einstellen bzw. ein gezielter Verlauf der Konzentration erzeugen.

In einem dritten, optionalen Verfahrensschritt, der ein Kristallisationsschritt ist, wird das bioaktive Oberflächenmaterial kristallisiert. Bei dem bioaktiven Oberflächenmaterial handelt es sich hierzu um ein kristallisierbares Material. Beispielsweise kann es sich um Hydroxylapatit (HAp), aber auch andere biokompatible Materialien, die zur Bildung einer bioaktiven Oberfläche geeignet sind handeln. Die Kristallisation wird insbesondere innerhalb des Oberflächenbereichs und/oder im Bereich bzw. ausgehend von der ersten Oberfläche gestartet. Um die Kristallisation zu starten wird eine Sättigungs-, vorzugsweise eine Übersättigungskonzentration des bioaktiven Oberflächenmaterials innerhalb des Lösungsmittels im gewünschten Bereich erzeugt. Durch Regelung der Umgebungsparameter innerhalb der abgeschlossenen Umgebung wird die Lösung insbesondere derart gesteuert, dass sich ein flüssiger Lösungsfilm auf der ersten Oberfläche des Ausgangskörpers bildet. Durch Verdunstung und/oder Verdampfung und/oder Trocknung des Lösungsmittels wird die Kristallisation gestartet, wodurch sich eine feste Kristallschicht auf der ersten Oberfläche des Ausgangskörpers bildet. Je nach eingestellter Konzentration bzw. eingestelltem Konzentrationsverlauf kristallisiert das bioaktive Oberflächenmaterial innerhalb der Poren des Oberflächenbereichs bzw. bis zur gewünschten Infiltrationstiefe innerhalb der Poren des Ausgangskörpers. Durch Kristallisationskeime, sog. Impfkristalle innerhalb der Poren im Oberflächenbereich kann die Kristallbildung beschleunigt und auch gezielt gesteuert werden.

Aufgrund der Kristallisation des bioaktiven Oberflächenmaterials innerhalb der Poren des Ausgangskörpers, insbesondere innerhalb der Poren des Oberflächenbereichs wird ein Form- und/oder Kraft- und/oder Reibschluss des bioaktiven Oberflächenmaterials mit dem Ausgangskörper erzielt. Je nach zuvor eingestellter Infiltrationstiefe, wird ein mechanisch fester Verbund zwischen der kristallinen Schicht und dem Ausgangskörper erzeugt, sodass ein Abplatzen der Schicht, d.h. der bioaktiven Oberflächenbeschichtung bzw. eine Beschädigung des Implantatrohlings vermieden werden kann.

In einem optionalen Kristallwachstumsschritt, wachsen Kristalle des bioaktiven Oberflächenmaterials, ausgehend von der Porosität, d.h. von den Poren des Oberflächenbereichs und/oder ausgehend von der ersten Oberfläche zu einer kristallinen Schicht. Die kristalline Schicht bedeckt die erste Oberfläche des Ausgangskörpers vollständig oder bedeckt zumindest Teile der ersten Oberfläche des Ausgangskörpers. Der Kristallwachstumsschritt erfolgt vorzugsweise als vierter Schritt im Anschluss an den Kristallisationsschritt, wobei die Schichtdicke der kristallinen Schicht erhöht wird. Die bevorzugte Schichtdicke der kristallinen Schicht beträgt nach der End- bzw. Dichtsinterung durchschnittlich zwischen 0,10 - 250 µm, um ein Einheilen des Implantats in das umliegende Gewebe zu erleichtern bzw. zu beschleunigen. Es ist jedoch problemlos möglich eine Schichtdicke von bis zu 2000 µm zu erzeugen. Die Schichtdicke ist abhängig von der Kristallisationsdauer, d.h. von der Dauer des Kristallwachstumsschritts, der Konzentration des bioaktiven Oberflächenmaterials in der Lösung sowie von der Art des verwendeten Lösungsmittels. Im Gegensatz zu den aus dem Stand der Technik bekannten Verfahren wird die kristalline, bioaktive Oberflächenbeschichtung nicht von außen auf den Implantatkörper aufgebracht, sondern aus dessen Innerem heraus, mittels Kristallwachstum gebildet, da sich das bioaktive Oberflächenmaterial bereits in den Poren des Ausgangskörpers befindet.

Nach einer bevorzugten Ausführung des Verfahrens erfolgt der Kristallwachstumsschritt kontinuierlich, wobei der Beladungsschritt zumindest teilweise zeitgleich ausgeführt wird. Kontinuierlich bedeutet insbesondere, dass die Beladung und das Kristallwachstum zumindest zeitweise gleichzeitig erfolgen, wobei mittels des Beladungsreservoirs kontinuierlich Lösungsmittel mit dem bioaktiven Oberflächenmaterial der Beladungsfläche zugeführt und von dieser aufgenommen wird. Im kontinuierlichen Verfahren wird das Lösungsmittel inklusive dem bioaktiven Oberflächenmaterial und/oder den chemischen Stoffen zur Beeinflussung der physikalischen Eigenschaften bzw. der Farbgebung oder auch reines Lösungsmittel zunächst dem Beladungskörper, der in dem Beladungsreservoir angeordnet ist bzw. den entsprechenden Beladungszonen des Beladungskörpers zugeführt. Anschließend wird dieses durch Parallel- oder Reihenbeladung von der Beladungsfläche aufgenommen. Durch Regelung der Umgebungsparametergradienten innerhalb der abgeschlossenen Umgebung wird das Lösungsmittel mit dem entsprechenden Solvat zu dem gewünschten Bereich, insbesondere dem Oberflächenbereich gesteuert. Ausgehend von den Poren des Oberflächenbereichs bzw. von der Oberfläche des Implantatrohlings wird das Lösungsmittel verdampft und/oder verdunstet und/oder getrocknet wobei das bioaktive Oberflächenmaterial von der Oberfläche ausgehend eine feste, kristalline Schicht bildet. Das verdampfte und/oder verdunstete und/oder getrocknete Lösungsmittel wird aus der abgeschlossenen Umgebung in die Außenumgebung abgeführt und kontinuierlich durch Beladung über die Beladungsfläche aus dem Beladungsreservoir ersetzt. Das Flüssigkeitsvolumen des verdampften und/oder verdunsteten Lösungsmittels entspricht dabei dem Volumen des Lösungsmittels inklusive dem darin gelösten bioaktiven Oberflächenmaterial entspricht, welches von der Beladungsfläche aufgenommen wird.

In einem optionalen Porenbildungsschritt, werden die Kristalle und/oder die kristalline Schicht des bioaktiven Oberflächenmaterials zur Bildung von Poren und/oder zur Erhöhung der Oberflächenrauigkeit einer Wärmebehandlung unterzogen. Die mittels des bioaktiven Oberflächenmaterials gebildete bioaktive Oberflächenbeschichtung weist bereits eine ausreichende Rauigkeit auf, die ein Einheilen des umliegenden Gewebes, insbesondere des Kieferknochens in die Poren gewährleistet. Um die Rauigkeit und/oder die Anzahl der Poren bzw. die Porengröße zu erhöhen, kann der Implantatrohling nach Ausbildung der bioaktiven Oberflächenbeschichtung einer Wärmebehandlung, bei einer Temperatur in einem Bereich von 500 - 800°C unterzogen werden. Vorzugsweise liegt der Durchmesser der Poren der kristallinen Oberflächenbeschichtung in einem Bereich zwischen 50 und 200 µm.

Vorteilhafterweise kann eine Morphologie und/oder Struktur und/oder Oberfläche und/oder Porosität, insbesondere Anzahl und Größe der Poren, der kristallinen Schicht und/oder der Kristalle des bioaktiven Oberflächenmaterials mittels des Lösungsmittels, d.h. durch die Wahl des Lösungsmittels während des Verfahrens beeinflusst werden.

Als Lösungsmittel eignet sich beispielsweise Wasser, insbesondere destilliertes Wasser. Durch die Verwendung von Ethanol können flachere und dichtere kristalline Schichten erzeugt werden. Ethanol weist eine höhere Verdampfungs- bzw. Verdunstungsgeschwindigkeit als Wasser auf wodurch die Kristallbildung schneller verläuft. Eine höhere Oberflächenrauigkeit kann z. B. durch die Verwendung von Essigsäure als Lösungsmittel erzielt werden. Es ist aber auch die Verwendung anderer Lösungsmittel zur Beeinflussung der Kristallbildung denkbar.

In einem optionalen Wärmeausdehnungskoeffizienten-Ausgleichsschritt, wird der Ausgangskörper mit einer Ausgleichslösung, die Zirkonium und/oder Calcium und/oder Cer aufweist, gespült. Der Wärmeausdehnungskoeffizient (WAK) gibt an um welchen Faktor sich ein Körper pro Temperaturänderung (in Kelvin) ausdehnt. Durch eine Spülung des Ausgangskörpers, mit einer Lösung, die insbesondere Zirkon- und/oder Calciumnitrate enthält, lässt sich ein WAK-Verlauf zwischen dem Oberflächenbereich und dem Kernbereich des Ausgangskörpers bzw. des Implantatrohlings einstellen. Unter einer Spülung wird eine Beladung mit der Lösung sowie eine anschließende Steuerung der Verteilung der Lösung innerhalb des Ausgangskörpers verstanden. Vorzugsweise wird ein Wärmeausdehnungskoeffizienten-Verlauf vom Kernbereich zur Oberfläche, bevorzugt von WAK = 14/K hin zu 9/K eingestellt. Zur gezielten Verteilung der Ausgleichslösung kann sich zu Nutze gemacht werden, dass ein Teil des Porenvolumens des Ausgangskörpers bereits mit dem biokompatiblen Oberflächenmaterial befüllt ist. Der Ausgangskörper weist einen abnehmenden Konzentrationsverlauf des biokompatiblen Oberflächenmaterials ausgehend von dessen Oberfläche hin zu dessen Kernbereich auf, wodurch eine anschließende Spülung des Ausgangskörpers mit der Ausgleichslösung zu einem umgekehrten Konzentrationsverlauf der Ausgleichslösung führt. Durch den Wärmeausdehnungskoeffizienten-Ausgleichsschritt wird die Wahrscheinlichkeit des Abplatzens der bioaktiven, kristallinen Oberflächenschicht von der Oberfläche des Ausgangskörpers während einer anschließenden Wärmebehandlung und/oder einem Dicht- bzw. Endsinterschritt weiter verringert. Durch einen stetigen Verlauf des Wärmeausdehnungskoeffizienten wird die wärmebedingte Volumenausdehnungsdifferenz zwischen dem Kernbereich, dem Oberflächenbereich und der bioaktiven, kristallinen Oberflächenbeschichtung ausgeglichen.

Nach einer vorteilhaften Verfahrensvariante wird eine Aufnahmekapazität des Ausgangskörpers oder eine Aufnahmekapazität einzelner Bereiche des Ausgangskörpers durch eine Beladung mit reinem Lösungsmittel gesteuert. Insbesondere kann beispielsweise der Kernbereich des Ausgangskörpers vor und/oder während der Beladung mit der Lösung, die das biokompatible Oberflächenmaterial enthält gezielt mit reinem Lösungsmittel, zum Beispiel Ethanol und/oder Essigsäure und/oder destilliertem Wasser beladen werden, um ein Eindringen des biokompatiblen Oberflächenmaterials in die Poren des Kernbereichs zu vermeiden und/oder zu verringern. Alternativ kann die Aufnahmekapazität der Poren einzelner Bereiche des Ausgangskörpers durch Gaseinschlüsse, insbesondere Lufteinschlüsse selektiv gesteuert werden.

Gemäß einer optionalen Verfahrensvariante ist mindestens ein Trägermaterial des porösen Ausgangskörpers kristallisierbar, wobei die Kristallbildung des kristallisierbaren Trägermaterials nicht vollständig abgeschlossen ist. Bei dem kristallisierbaren Trägermaterial kann es sich insbesondere um Zirkonoxid oder andere für die Dentaltechnik geeignete Keramiken oder Metalle bzw. Metalllegierungen handeln.

In einem optionalen Gefriertrocknungsschritt wird das bioaktive Oberflächenmaterial in den Poren der gewünschten Bereiche des Ausgangskörpers fixiert. Auf diese Weise kann insbesondere eine Rückdiffusion des bioaktiven Oberflächenmaterials in das Innere des Ausgangskörpers behindert werden. Der Gefriertrocknungsschritt findet vorzugsweise unter Vakuum statt.

In einem optionalen Sinterschritt wird ein Stoffschluss und/oder ein chemischer Verbund zwischen dem bioaktiven Oberflächenmaterial und dem mechanisch belastbaren Trägermaterial erzeugt. Durch einen Sintervorgang, insbesondere im Bereich von Temperaturen um 1450 °C, verschmilzt das bioaktive Oberflächenmaterial, welches in den Poren des Ausgangskörpers angeordnet ist, mit dem mechanisch belastbaren Trägermaterial wodurch der Stoffschluss und/oder chemische Verbund erzielt wird. Insbesondere sind sowohl das bioaktive Oberflächenmaterial bzw. das mechanisch belastbare Trägermaterial kristallisierbar oder besitzen zumindest kristallisierbare Komponenten wodurch eine gemeinsame Kristallstruktur gebildet und die Verbundstabilität erhöht wird. Bei dem optionalen Sinterschritt kann es sich auch um ein End- bzw. Dichtsintern des Implantatrohlings handeln, welches diesen zum fertigen Implantat werden lässt.

In einer alternativen Ausführung des Verfahrens umfasst der poröse Ausgangskörper ein inertes Metall oder besteht vorzugsweise aus einem inerten Metall, insbesondere einem Dentalmetall. Inerte Metalle wie bspw. Titan eignen sich besonders gut zur Herstellung von Implantaten, da diese an ihrer Oberfläche eine Oxydschicht bilden, wodurch umliegende Knochen und Gewebe nicht abgestoßen werden. Unter Dentalmetall werden Metalle verstanden, die für die Herstellung von Zahnersatz oder Implantaten im Zahnbereich geeignet sind.

Das erfindungsgemäßen Verfahren wird vorzugsweise zur Herstellung von Implantatrohlingen aus Ausgangskörpern, die bereits die gewünschte Implantatform aufweisen verwendet. Es ist aber ebenfalls möglich, die Verteilung des biokompatiblen Oberflächenmaterials innerhalb eines, zum Beispiel plattenförmigen Rohlings zu steuern, diesen mittels einer CAD/CAM-Fräsmaschine in die gewünschte Raumform zu Fräsen und anschließend dem Kristallbildungs- und/oder dem Kristallwachstumsschritt zu unterziehen. Auf diese Weise können aus einem Ausgangskörper mehrere Implantatrohlinge hergestellt werden.

Während der Beladung mit dem und/oder der Steuerung des bioaktiven Oberflächenmaterials innerhalb des Ausgangskörpers kann gleichzeitig eine Beladung und/oder Steuerung mit verschiedenen chemischen Stoffen zur Beeinflussung der physikalischen Eigenschaften des Implantatrohlings und/oder zum Einfärben bzw. zur Erzeugung eines zweidimensionalen oder dreidimensionalen Farbverlaufs erfolgen.

Ferner wird ein Implantatrohling, insbesondere nach dem erfindungsgemäßen Verfahren hergestellt, beschrieben. Der Implantatrohling ist zur Herstellung eines bioaktiven Implantats, insbesondere eines Dentalimplantats, geeignet und weist mindestens einen ersten Bereich, der ein Oberflächenbereich ist und einen zweiten Bereich, der ein Kernbereich ist auf. Der Oberflächenbereich umfasst ein bioaktives Oberflächenmaterial und der Kernbereich ein mechanisch belastbares Trägermaterial. Ausgehend von einer ersten Oberfläche umfasst der Implantatrohling eine kristalline Schicht, die das bioaktive Material aufweist. Die kristalline Schicht ist form- und/oder kraftschlüssig mit dem Oberflächenbereich verbunden, wobei das bioaktive Oberflächenmaterial innerhalb von Poren des Oberflächenbereichs angeordnet ist. Zwischen dem bioaktiven Oberflächenmaterial und dem mechanisch belastbaren Trägermaterial kann zusätzlich ein Stoffschluss und/oder ein chemischer Verbund bestehen.

Eine Infiltrationstiefe der kristallinen Schicht in die Poren des Oberflächenbereichs beträgt mindestens 50 µm, vorzugsweise mindestens 75 µm und besonders bevorzugt mindestens 100 µm. Je höher die Infiltrationstiefe des biokompatiblen Oberflächenmaterials, desto stärker ist die mechanische und/oder stoffschlüssige und/oder chemische Verbindung zu dem Trägermaterial, wodurch ein Abplatzen der kristallinen Schicht verhindert wird. Vorzugsweise nimmt die Menge des bioaktiven Oberflächenmaterials, welches innerhalb der Poren angeordnet ist, ausgehend von der Oberfläche des aus Körpers hin zu dessen Kernbereich stetig und/oder gleichmäßig ab.

Weitere Einzelheiten, Merkmale, Merkmals(unter)kombinationen und Wirkungen auf der Basis der Erfindung ergeben sich aus der nachfolgenden Beschreibung bevorzugter, beispielhafter Ausführungsformen der Erfindung sowie aus den Zeichnungen. Diese zeigen in:
Figur 1 eine schematische Darstellung eines Dentalimplantats, welches zur Aufnahme eines Zahnersatzes in den Kieferknochen eines Patienten eingeschraubt ist,
Figur 2 ein Flussdiagramm zur schematischen Darstellung des Ablaufs eines beispielhaften, erfindungsgemäßen Herstellungsverfahrens,
Figur 3 eine schematische Darstellung eines Dentalimplantatrohlings, der für das erfindungsgemäße Herstellungsverfahren in einer abgeschlossenen Umgebung angeordnet ist,
Figur 4 schematisch das Wachstum einer kristallinen, bioaktiven Oberflächenbeschichtung,
Figur 5 mikroskopische Aufnahmen einer kristallinen, bioaktiven Oberflächenbeschichtung gemäß der Wachstumsschritte aus Figur 4,
Figur 6 eine mikroskopische Aufnahme einer kristallinen Oberflächenbeschichtung mit isolierten Bereichen,
Figur 7 eine schematische Darstellung einer alternativen Ausführungsvariante des erfindungsgemäßen Verfahrens.

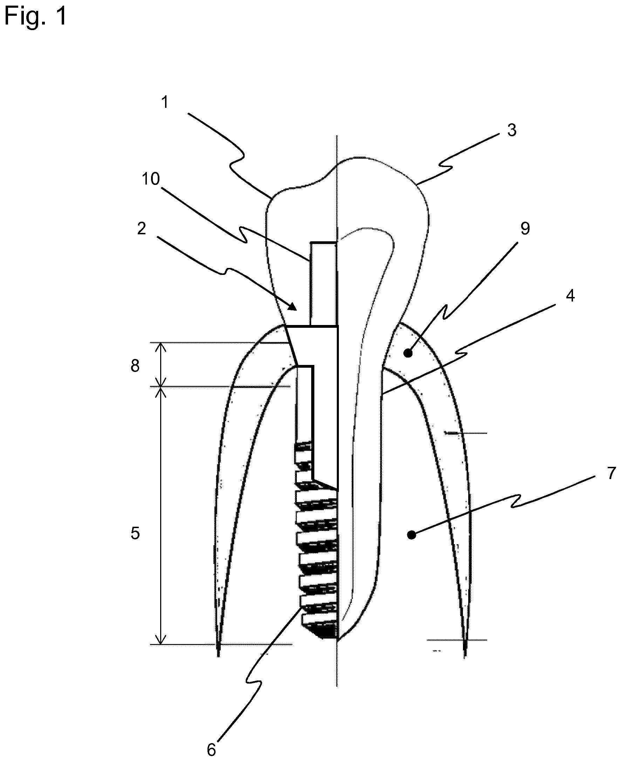

In Figur 1 ist ein Zahnersatz 1, insbesondere eine Krone, mit einem darunter angeordneten Dentalimplantat 2 in Gegenüberstellung zu einem Zahn 3 mit darunterliegender Zahnwurzel 4 schematisch dargestellt. Das Dentalimplantat 2 umfasst einen Implantatkörper 5, der mit einem Außengewinde 6 versehen ist und zum Einschrauben in den Kieferknochen 7 eines Patienten vorgesehen ist. Oberhalb des Implantatkörpers 5 schließt sich ein Übergangsabschnitt 8 an, der je nach Ausführung des Dentalimplantats 2 einstückig mit diesem ausgeführt sein kann oder, wie hier dargestellt, in den Implantatkörper 5 eingeschraubt ist. Der Übergangsabschnitt 8 ist innerhalb des Zahnfleischs 9 des Patienten angeordnet. An den Übergangsabschnitt 8 schließt sich das sogenannte Abutment 10 an, welches aus dem Zahnfleisch 9 des Patienten herausragt und zur Aufnahme des Zahnersatzes 1 ausgebildet ist. Die Oberfläche des Implantatkörpers 5 ist vorzugsweise als bioaktive Oberfläche ausgebildet, sodass der umliegende Kieferknochen 7 in den Implantatkörper 5 einheilen kann. Der Übergangsabschnitt 8, welcher innerhalb des Zahnfleischs 9 angeordnet ist, sollte eine möglichst glatte, nicht poröse Oberfläche aus einem biokompatiblen Material aufweisen, um ein Einwachsen des Zahnfleischs sowie das Ansiedeln und die Vermehrung von Bakterien zu verhindern.

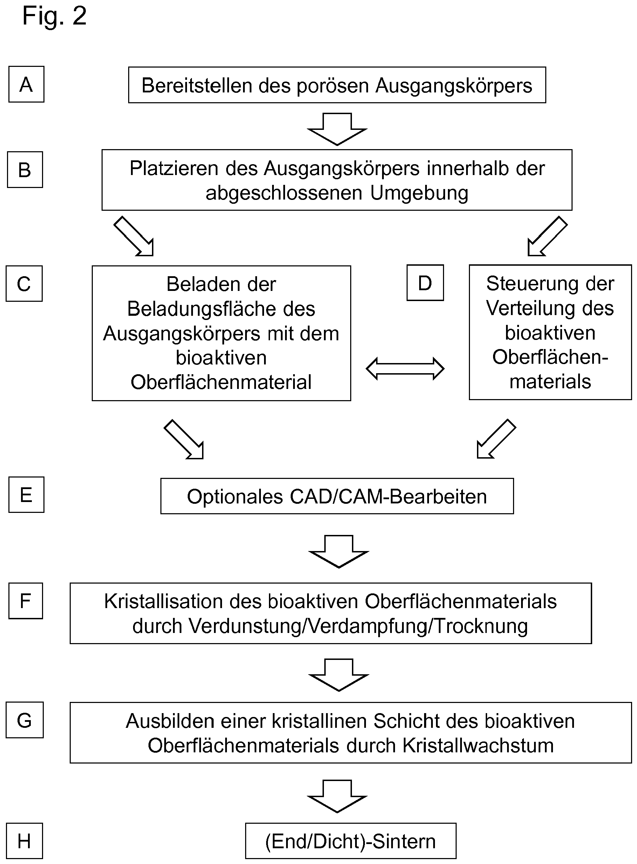

In Figur 2 ist ein erfindungsgemäßer Verfahrensablauf beispielhaft anhand eines Flussdiagramms dargestellt. In einem ersten Schritt (A) wird ein poröser Ausgangskörper, zum Beispiel ein zylindrischer Zirkonoxid-Keramikrohling mit einem Durchmesser von 16,5 mm, einer Höhe von 16 mm und einer Gesamtmasse von 10,32 g bereitgestellt. Der Ausgangskörper wird in einem zweiten Schritt (B) in einer abgeschlossenen Umgebung platziert. Hierbei kann es sich, um eine zylinderförmige Kammer handeln. Eine erste Oberfläche des Ausgangskörpers, an welcher die Bildung einer bioaktiven Oberflächenbeschichtung erfolgen soll, ist vollständig innerhalb der abgeschlossenen Umgebung angeordnet, wobei eine zweite Oberfläche oder mindestens ein Teil der zweiten Oberfläche, bei der es sich um eine Beladungsfläche handelt außerhalb der abgeschlossenen Umgebung angeordnet ist. In einem dritten Schritt (C) wird die Beladungsfläche des Ausgangskörpers mit einer Lösung eines bioaktiven Oberflächenmaterials beladen. Bei der Lösung handelt es sich um ein Hydroxylapatit-Sol, welches durch Lösen von 2,5 g Calciumnitrat sowie 1,5 g Triethylphosphat in 40 g Ethanol hergestellt wurde. Die Prekursoren des Sols liegen im Verhältnis Ca/PO₄ = 10/6 vor. Die maximale Aufnahmekapazität der Poren des Zirkonoxid-Keramikrohlings beträgt 1,55 g des Solvats. Zeitgleich mit oder nachfolgend auf die Beladung (C) erfolgt eine Steuerung der Verteilung (D) des bioaktiven Oberflächenmaterials, wobei die Lösung innerhalb des Oberflächenbereichs eine höhere Konzentration aufweist als innerhalb des Kernbereichs. Zur Steuerung wird mindestens ein Umgebungsparameter, insbesondere die Luftfeuchtigkeit und/oder der Druck und/oder die Temperatur zur Erzeugung von Umgebungsparametergradienten geregelt. Verzugsweise wird in der Kammer eine Temperatur von 25 °C und eine Luftfeuchtigkeit von 30 % eingestellt, wodurch sich ein Temperatur- bzw. Luftfeuchtigkeitsgradient zwischen der ersten Oberfläche und der Beladungsfläche ergibt. In einem optionalen CAD/CAM-Bearbeitungsschritt (E) kann der zylinderförmige Keramikrohling in die gewünschte Raumform gefräst werden. Die Verteilung des bioaktiven Oberflächenmaterials kann vorher derart gesteuert sein, dass sich Volumenbereiche mit erhöhter Konzentration des bioaktiven Oberflächenmaterials innerhalb des Ausgangskörpers ergeben. Vorzugsweise werden diese Bereiche durch die anschließende Fräsbearbeitung zu Außen- bzw. Oberflächen eines Implantatrohlings. In einem Kristallisationsschritt (F) wird das bioaktive Oberflächenmaterial kristallisiert, wobei die Konzentration der Lösung innerhalb des Oberflächenbereichs durch Verdunstung und/oder Verdampfung und/oder Trocknung bis zu einer Sättigungskonzentration erhöht wird. Während eines Kristallwachstumsschritts (G) wird eine kristalline Schicht des bioaktiven Oberflächenmaterials ausgehend von der ersten Oberfläche des Ausgangskörpers gebildet. Je nach gewünschter Schichtdicke und -struktur der kristallinen Oberflächenbeschichtung erfolgt die Wachstumsphase beispielsweise drei Tage innerhalb der abgeschlossenen Kammer. Um das Kristallwachstum zu beenden wird der Ausgangskörper über weitere zwei Tage mit reinem Lösungsmittel, vorzugsweise Ethanol gespült um Rückstände des Hydroxylapatit-Sols, insbesondere aus dem Kernbereich des Ausgangskörpers zu entfernen. Wurde der Ausgangskörper zuvor mittels Parallelbeladung beladen, d.h. mit einem Beladungskörper, der mehrere nebeneinander angeordnete Beladungszonen aufweist, so kann die Notwendigkeit einer Spülung vermieden werden. Mittels der Parallelbeladung ist es möglich den Kernbereich mit einer Lösung, die wiederum kein bioaktives Oberflächenmaterial enthält zu beladen, während dem Oberflächenbereich gleichzeitig bioaktives Oberflächenmaterial zugeführt wird. Auf diese Weise bleibt der Kernbereich während des Verfahrens und auch nach Fertigstellung des Dentalimplantats vollständig frei von HAp-Rückständen, da die Poren des Kernbereichs durch Aufnahme der HAp-freien Lösung "blockiert" sind. Anschließend kann eine dreitägige Trocknung bei 25 °C und 20 % Luftfeuchtigkeit erfolgen. Durch Erhöhung der Hydroxylapatit-Sol-Konzentration wird das Kristallwachstum beschleunigt, durch Erhöhung der Dauer des Kristallwachstumsschritt kann eine höhere Schichtdicke eingestellt werden. Eine andere Möglichkeit hierzu ist die Erhöhung der Trocknungsdauer auf 30 Tage bei 22 °C und 40% Luftfeuchtigkeit. Durch ein abschließendes End- bzw. Dichtsintern wird der bioaktiv beschichtete Implantatrohling zum Implantat fertiggestellt. Das Sintern findet in einem Sinterofen in einem Bereich von 1300 -1600, gängigerweise bei etwa 1450 °C (hersteller- bzw. materialabhängig) über 4 Stunden statt. Durch den Sintervorgang findet eine Kalzifizierung statt, wodurch ein synthetisches Hydroxylapatit entsteht. Vorteilhaft ist eine abschließende Wärmebehandlung bei 150 - 200 °C über weitere 4 Stunden. Über weitere 10 - 50 Stunden sollte eine hydrothermale Wärmebehandlung bei 150 - 200°C zur Umwandlung von Tricalciumphosphat (TCP) zu Hydroxylapatit (HAp) stattfinden.

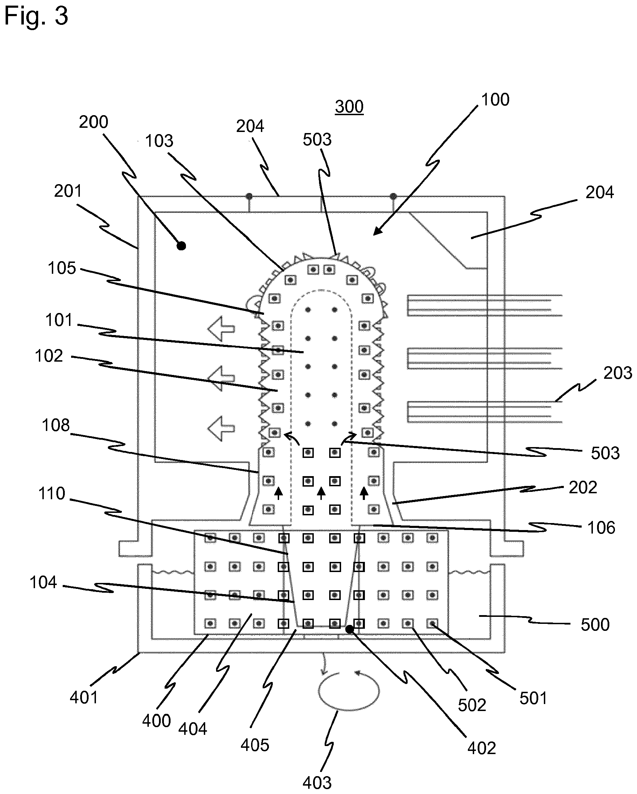

Figur 3 zeigt eine schematische Darstellung eines rotationssymmetrischen Dentalimplantatrohlings bzw. Ausgangskörpers 100, der in einer abgeschlossenen Umgebung 200 angeordnet ist. Im vorliegenden Beispiel ist die abgeschlossene Umgebung 200 als Kammer 201 ausgeführt, es könnte sich aber beispielsweise auch um einen Schrank oder Raum zur Aufnahme mehrerer oder einer Vielzahl von Dentalimplantatrohlingen 100 handeln. Der Dentalimplantatrohling 100 ist einstückig ausgeführt und weist einen Implantatkörper 105, einen Übergangsabschnitt 108 sowie ein Abutment 110 auf. Weiterhin umfasst der Dentalimplantatrohling 100 einen Kernbereich 101 sowie einen Oberflächenbereich 102, der sich ausgehend von einer ersten Oberfläche 103 in Richtung des Kernbereichs 101 erstreckt und vorzugsweise nahtlos in diesen übergeht. Die erste Oberfläche 103 umfasst die Mantelfläche sowie die Außenfläche des halbkugelförmigen Endabschnitts des Dentalimplantatrohlings 100 und ist vollständig innerhalb der abgeschlossenen Umgebung 200 angeordnet. Der Übergangsabschnitt 108 schließt sich an den Implantatkörper 105 an und endet im Abutment 110, welches aus der abgeschlossenen Umgebung herausragt. Im Bereich des Übergangsabschnitt 108 ist die abgeschlossene Umgebung mittels einer Isolierung 202 (nicht dargestellt), zum Beispiel einer Silikondichtung oder ähnlichem gegenüber der Außenumgebung 300 abgedichtet.

Die Außenfläche 104 des Abutments 110 sowie die, in Richtung des Abutments weisende Auflagefläche 106 des Übergangsabschnitts 108 sind jeweils als zweite Oberfläche bzw. Beladungsfläche angeordnet. Das Abutment 110 wird hierzu von einem Beladungskörper 400, der sich in einem Beladungsreservoir 401 befindet, aufgenommen. Der Beladungskörper 400 ist mit einer Ausnehmung 402 versehen, deren Form zur Aufnahme des Abutments 110 an dessen Abmessungen angepasst ist. Bei der Verwendung des Beladungskörpers 400 handelt es sich um eine optionale Ausführung des Verfahrens. Alternativ können die Beladungsflächen, d.h. die Außenfläche 104 und/oder die Auflagefläche 106 auch direkt innerhalb des Beladungsreservoirs 401 angeordnet sein. Der Beladungskörper 400 weist eine erste Beladungszone 404 sowie eine zweite Beladungszone 405 auf, die wie zuvor beschrieben, zur Aufnahme unterschiedlicher Lösungen oder gleicher Lösungen mit unterschiedlicher Konzentration geeignet sind. Gemäß einer ebenfalls alternativen und nicht dargestellten Ausführungsform ist der Dentalimplantatrohling 100 zweiteilig ausgeführt. In dieser Variante ist das Abutment 110 aus dem Implantatkörper 105 herausgeschraubt, sodass nur die Auflagefläche 106 auf dem Beladungskörper 400 aufliegt. Eine Ausnehmung 402 zur Aufnahme des Abutments 110 ist folglich nicht erforderlich. Anstelle der Ausnehmung 402 könnte beispielsweise die zweite Beladungszone 405 angeordnet sein, um eine Parallelbeladung der Auflagefläche 106 zu ermöglichen. Auf diese Weise könnte der Kernbereich 101 zum Beispiel mit reinem Lösungsmittel 501 oder mit einer Lösung 500, die insbesondere Cer und/oder Calcium und/oder Zirkonium, jedoch kein bioaktives Oberflächenmaterial 502 aufweist, beladen werden, während gleichzeitig der Oberflächenbereich 102 über dieselbe Auflagefläche 106 mit einer Lösung 500, die ein bioaktives Oberflächenmaterial 502 umfasst, beladen wird. So wird sichergestellt, dass der Kernbereich 101 frei von bioaktivem Oberflächenmaterial 502 bleibt.

Das Beladungsreservoir 401 ist mit einem festgelegten Volumen einer Lösung 500 befüllt, die ein Lösungsmittel bzw. Solvens 501, insbesondere destilliertes Wasser, Ethanol und/oder Essigsäure sowie ein bioaktives Oberflächenmaterial bzw. Solvat 502 wie beispielsweise Hydroxylapatit umfasst. Das Beladungsreservoir 401 ist gegen die Außenumgebung 300 abgedichtet, um eine Verdunstung des Lösungsmittels, die zu einer Änderung der Konzentration der Lösung führen würde zu verhindern. Alternativ und nicht dargestellt können verschließbare Zu- bzw. Abflüsse vorgesehen sein um ein kontinuierliches Nachfüllen der Lösung 500 zu ermöglichen bzw. um die Konzentration der Lösung 500 bei Bedarf ändern zu können. Während des Beladungsschritts wird die Lösung 500 mittels des Beladungskörpers 400, der eine oder mehrere Beladungszonen 404, 405 aufweist, den Beladungsflächen 104, 106 zugeführt. Die Lösung wird von den Beladungsflächen 104, 106 aufgrund der Kapillarkraft und/oder Konzentrationsunterschieden bzw. Umgebungsparametergradienten, die innerhalb der abgeschlossenen Umgebung 200 gegenüber dem Beladungsreservoir 401 einstellbar sind, aufgenommen. Zur Beendigung des Beladungsschritts werden das Beladungsreservoir 401 und der Beladungskörper 400 entfernt.

Während des Verteilungsteuerungsschritts, der zeitgleich oder nachfolgend auf den Beladungsschritt erfolgen kann wird eine Konvektionsströmung 503 innerhalb der Poren des Dentalimplantatrohlings 100 erzeugt. Hierzu werden innerhalb der abgeschlossenen Umgebung 200 Umgebungsparametergradienten, insbesondere durch Änderung der Temperatur, des Drucks und/oder der Luftfeuchtigkeit erzeugt. Die Kammer 201 weist hierzu geeignete Mittel auf. Die Lösung wird vorzugsweise zur ersten Oberfläche 103 bzw. zu dem Oberflächenbereich 102 des Dentalimplantatrohlings 100 gesteuert.

Um die Kristallisation des bioaktiven Materials 502 zu starten wird eine Trocknung und/oder Verdunstung des Lösungsmittels 501 an der ersten Oberfläche 103 des Dentalimplantats 100 durch (Heiß-)Luftdüsen 203, erzielt. Die Temperatur sowie der Volumenstrom der Luftzufuhr können unabhängig voneinander geregelt werden. Um die zugeführte Luft sowie das verdunstete Lösungsmittel 501 abzuführen weist die Kammer 201 eine Entlüftungsklappe 204 auf. Alternativ oder zusätzlich können auch andere Entlüftungsmittel, wie Ventile, Abzüge oder ähnliches vorgesehen sein. Durch Trocknung und/oder Verdunstung wird die Konzentration des gelösten bioaktiven Oberflächenmaterials 502 an der ersten Oberfläche 103 sowie innerhalb der Poren des Oberflächenbereichs 102 bis zu einer Sättigungskonzentration erhöht. Innerhalb der Poren des Oberflächenbereichs 102 entstehen erste Kristalle, die ausgehend von der ersten Oberfläche 103 eine kristalline Schicht bzw. eine kristalline Oberflächenbeschichtung 503 bilden. Um eine möglichst gleichmäßige Luftzufuhr mittels der (Heiß-) Luftdüsen zu gewährleisten, ist das Beladungsreservoir 401 auf einem Drehteller 403 drehbar gelagert, wodurch der Dentalimplantatrohling 100 innerhalb der Kammer 200 rotierbar ist.

In einem anschließenden Kristallwachstumsschritt wächst die kristalline Oberflächenbeschichtung 503 bis zur gewünschten Schichtdicke. Während des Kristallwachstumsschritts kann weiterhin eine Beladung mit Lösung 500, eine Steuerung der Verteilung der Lösung 500 durch Regelung von Umgebungsparametergradienten sowie eine Trocknung und/oder Verdunstung des Lösungsmittels 501 an der ersten Oberfläche 103 des Dentalimplantatrohlings 100 erfolgen. Die Dauer des Kristallwachstumsschritts hängt von der gewünschten Schichtdicke sowie der Menge an bioaktivem Oberflächenmaterial 502 mit dem der Dentalimplantatrohling 100 beladen wurde bzw. beladen wird ab. Eine in die Kammer integrierte UV-Lampe 204 trägt zur schnelleren Aushärtung der kristallinen Oberflächenbeschichtung 503 bei.

In einem optionalen Wärmeausdehnungskoeffizienten-Ausgleichsschritt wird der Dentalimplantatrohling 100 mit einer Cer und/oder Zirkonium und/oder Calcium enthaltenden Ausgleichslösung 500 beladen. Vorzugsweise wird hierbei die Außenfläche 104 des Abutments 110 als Beladungsfläche genutzt, um die Ausgleichslösung 500 gezielt in den Kernbereich 101 des Dentalimplantatrohlings einzubringen. Alternativ kann die Lösung auch mittels des Verteilungsteuerungsschritt in den Kernbereich eingebracht werden. Die Beladung mit der Ausgleichslösung 500 erfüllt zum einen den Zweck, den Kernbereich von Rückständen des bioaktiven Oberflächenmaterials 502 zu reinigen. Eine Reinigung des Kernbereichs kann auch durch Spülung mit reinem Lösungsmittel 501 vorgenommen werden. Zum anderen findet ein Wärmeausdehnungskoeffizientenausgleich statt, indem das Restvolumen der Poren mit Cer und/oder Zirkonium und/oder Calcium befüllt wird, wodurch ein Verlauf des Wärmeausdehnungskoeffizienten ausgehend von der ersten Oberfläche 103 hin zum Kernbereich 101 entsteht.

In einem abschließenden End- bzw. Dichtsinterschritt wird der Dentalimplantatrohling 100 zum fertigen Dentalimplantat bei Temperaturen um 1450 °C dichtgesintert. Hierbei erhält die kristalline Oberflächenbeschichtung 503 ihre abschließende Struktur, wobei die Schichtdicke durch den Sintervorgang abnimmt.

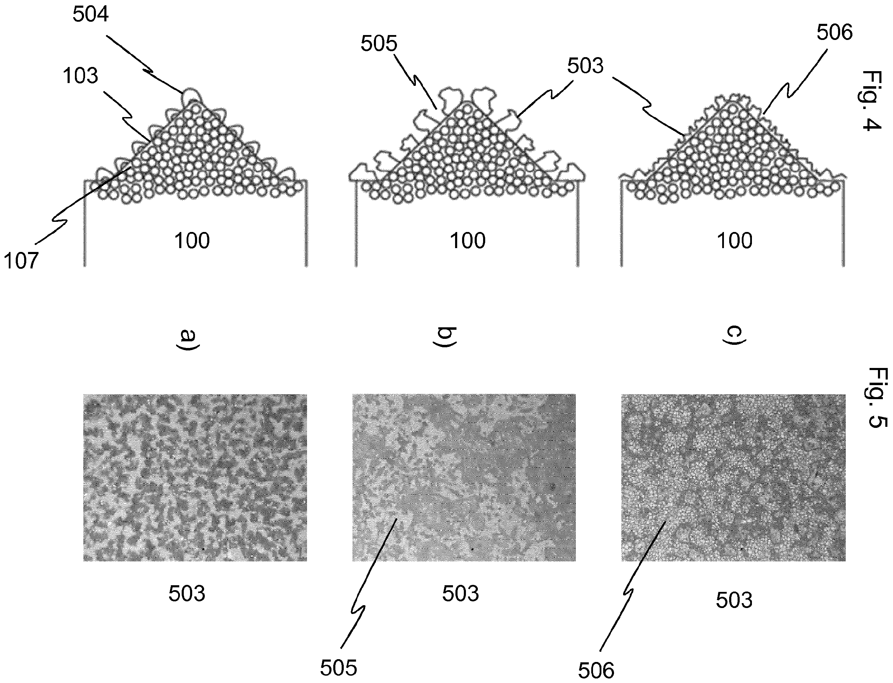

In Figur 4 ist schematisch der Ablauf der Ausbildung einer bioaktiven, kristallinen Oberflächenbeschichtung 503 von einer ersten Oberfläche 103 eines porösen Ausgangskörpers bzw. eines Dentalimplantatrohlings 100 ausgehend dargestellt. Figur 5 zeigt jeweils eine zugehörige, mikroskopische Aufnahme der kristallinen Oberflächenbeschichtung 503. Die Figuren 4a und 5a zeigen die Entstehung erster Kristallkeime, die sich aus den Poren 107, unterhalb kondensierter Lösungsmitteltropfen 504 herausbilden. Die Kristallkeime sind nach 3 Tagen Trocknung auf einer ersten Oberfläche 103 einer porösen Zirkonoxid-Keramik gewachsen. Die Zirkonoxid-Keramik wurde zuvor mit einem Hydroxylapatit-Sol (2,5 g Calciumnitrate, 1,5 g Triethylphosphat gelöst in 40 g Ethanol) beladen. Die Trocknung wurde in einer Vorrichtung gemäß Figur 2 bei 25 °C und 30 % Luftfeuchtigkeit durchgeführt. Durch kontinuierlichen Lösungsmittelentzug, insbesondere durch Trocknung und/oder Verdampfung und/oder Verdunstung (siehe Figuren 4b und 5b) für weitere 48 Stunden schreitet das Kristallwachstum voran, wodurch sich eine immer dichter werdende bioaktive, kristalline Oberflächenbeschichtung 503 herausbildet. Durch weiteres Erhöhen der Dauer des Wachstumsschritts kann eine Schichtdicke von bis zu 2000 µm ausgebildet werden. Zwischen den einzelnen Kristallen entstehen Kristallzwischenräume 505, die sich durch ein abschließendes Endsintern bei in etwa 1450 °C zur Oberflächenstruktur 506 der bioaktiven, kristallinen Oberflächenbeschichtung 503 verdichten (Figuren 4c und 5c). Zum Sintern wird der Implantatrohling 100 in einem Sinterofen platziert. Die Temperatur wird in Schritten von 3 °C pro Minute auf 1450 °C erhöht. Der Implantatrohling 100 verbleibt für ca. 2,5 Stunden bei dieser Temperatur im Sinterofen. Anschließend wird die Temperatur im Ofen in Schritten von 3° C pro Minute auf 200 °C abgekühlt.

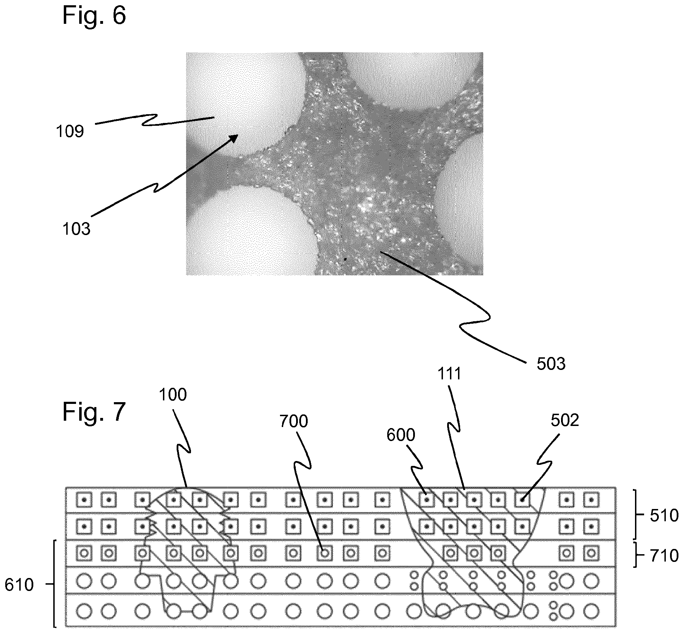

Figur 6 zeigt eine mikroskopische Aufnahme einer kristallinen Oberflächenbeschichtung 503, wobei einzelne Bereiche 109 der ersten Oberfläche 103 des Ausgangskörpers 100 gegen ein Kristallwachstum bzw. die Ausbildung der kristallinen Oberflächenbeschichtung 503 isoliert worden sind. Der Durchmesser eines isolierten Bereichs 109 beträgt in etwa 0,30 mm. Als Ausgangskörper wurde eine poröse Zirkonoxid-Keramik verwendet und mit einem Hydroxylapatit-Sol infiltriert bzw. beladen. Das verwendete Hydroxylapatit-Sol wurde aus 20 g Calciumnitrat, 20 g destilliertem Wasser sowie 12,8 g Triethylphosphat hergestellt. Zum Zeitpunkt der Aufnahme wurde der Ausgangskörper bereits 3 Tage in einer Vorrichtung gemäß Figur 2 einem Kristallwachstum bei 25 °C und 30 % Luftfeuchtigkeit unterzogen. Die Bereiche 109 wurden vor der Beladung durch Farbauftrag, mittels eines Druckers gegen Kristallwachstum isoliert. Die Isolierung der Bereiche 109 kann jedoch auch auf andere Weise, beispielsweise durch Beschichtung oder durch Aufbringen einer Folie erfolgen.

In Figur 7 ist eine alternative Verfahrensvariante schematisch dargestellt. Als Ausgangskörper wird beispielsweise ein zylindrischer, plattenförmiger, poröser Rohling aus Metall oder Keramik verwendet. Der Rohling wird gleichzeitig oder aufeinanderfolgend mit verschiedenen Lösungen beladen. Vorzugsweise enthält mindestens eine erste Lösung ein bioaktives, kristallisierbares Oberflächenmaterial 502, mindestens eine zweite Lösung einen chemischen Stoff zur Beeinflussung physikalischer Eigenschaften 600, zum Beispiel zur Beeinflussung der Härte sowie mindestens eine dritte Lösung farbgebende Komponenten 700. Die Verteilung der jeweiligen Lösungen kann so gesteuert werden, dass sich Bereiche mit einer erhöhten Konzentration an bioaktivem Material, Bereiche mit erhöhter Konzentration an Härte senkenden Stabilisatoren 610 sowie Bereiche mit erhöhter Konzentration an farbgebenden Komponenten 710, zum Beispiel zum Einfärben mit der Zahnfleischfarbe rosa herausbilden. Aus dem plattenförmigen Rohling kann anschließend ein Dentalimplantatrohling 100 und/oder ein Zahnersatzrohling oder auch ein. Kieferknochenersatzrohling 111 mittels CAD/CAM heraus gefräst und den übrigen Verfahrensschritten unterzogen werden.
- 1: Zahnersatz
- 2: Dentalimplantat
- 3: Zahn
- 4: Zahnwurzel
- 5: Implantatkörper
- 6: Außengewinde
- 7: Kieferknochen
- 8: Übergangsabschnitt
- 9: Zahnfleisch
- 10: Abutment
- 100: poröser Ausgangskörper/Dentalimplantatrohling
- 101: Kernbereich
- 102: Oberflächenbereich
- 103: erste Oberfläche
- 104: Außenfläche, Beladungsfläche
- 105: Implantatkörper
- 106: Auflagefläche, Beladungsfläche
- 107: Poren
- 108: Übergangsabschnitt
- 109: Bereich der ersten Oberfläche
- 110: Abutment
- 111: Zahnersatzrohling/Kieferknochenersatzrohling
- 200: Abgeschlossene Umgebung
- 201: Kammer
- 202: Isolierung
- 203: (Heiß-)Luftdüse
- 204: UV-Lampe
- 300: Außenumgebung
- 400: Beladungskörper
- 401: Beladungsreservoir
- 402: Ausnehmung
- 403: Drehteller
- 404: erste Beladungszone
- 405: zweite Beladungszone
- 500: Lösung
- 501: Lösungsmittel/Solvens
- 502: bioaktives Oberflächenmaterial/Solvat
- 503: kristalline Schicht/kristalline Oberflächenbeschichtung
- 504: Lösungsmitteltropfen
- 505: Kristallzwischenräume
- 506: Oberflächenstruktur
- 510: Bereich mit erhöhter Konzentration an bioaktivem Material
- 600: chemischer Stoff zur Beeinflussung physikalischer Eigenschaften
- 610: Bereich mit erhöhter Konzentration an Härte senkenden Stabilisatoren
- 700: farbgebende Komponenten
- 710: Bereich mit erhöhter Konzentration an farbgebenden Komponenten

## Patentansprüche

1. Verfahren zur Herstellung eines Implantatrohlings (100), insbesondere eines Dentalimplantatrohlings aus einem Ausgangskörper, welcher Implantatrohling (100) mindestens einen ersten Bereich, der ein Oberflächenbereich (102) ist und einen zweiten Bereich, der ein Kernbereich (101) ist umfasst, wobei der Oberflächenbereich (102) mindestens ein bioaktives Oberflächenmaterial (502) aufweist und sich von mindestens einer ersten Oberfläche (103) ausgehend in Richtung des Kernbereichs (101) erstreckt, und der Kernbereich (101) mindestens ein mechanisch belastbares Trägermaterial aufweist, **dadurch gekennzeichnet, dass** der Ausgangskörper eine Porosität zur Steuerung einer gezielten Verteilung des bioaktiven Oberflächenmaterials (502) innerhalb des Ausgangskörpers aufweist und in einem ersten Schritt, der ein Beladungsschritt ist, mit einer Lösung (500) des bioaktiven Oberflächenmaterials (502) beladen wird, und in einem zweiten Schritt, der ein Verteilungssteuerungsschritt ist, die Verteilung des bioaktiven Oberflächenmaterials (502) innerhalb des Ausgangskörpers durch eine Konvektionsströmung bewirkt wird, wobei eine Strömungsrichtung und -geschwindigkeit durch gezieltes Erzeugen von Umgebungsparametergradienten bezüglich verschiedener Oberflächen des Ausgangskörpers gesteuert werden, derart gesteuert wird, dass die Lösung (500) innerhalb des Oberflächenbereichs (102) eine höhere Konzentration aufweist als innerhalb des Kernbereichs (101), wobei die Steuerung durch Regelung eines oder mehrerer Umgebungsparameter in einer abgeschlossenen Umgebung (200), insbesondere durch Regelung der Luftfeuchtigkeit und/oder des Drucks und/oder der Temperatur erfolgt.

2. Verfahren nach Anspruch 1, **dadurch gekennzeichnet, dass** die Beladung des Ausgangskörpers mit der Lösung (500) des mindestens einen, bioaktiven Oberflächenmaterials (502) über mindestens eine zweite Oberfläche des Ausgangskörpers, die eine Beladungsfläche (104, 106) ist, erfolgt, wobei die Beladungsfläche (104, 106) eine von der ersten Oberfläche (103) verschiedene Oberfläche ist.

3. Verfahren nach Anspruch 2, **dadurch gekennzeichnet, dass** die Beladungsfläche (104, 106) zur Beladung des Ausgangskörpers mit dem bioaktiven Oberflächenmaterial (502) außerhalb der abgeschlossenen Umgebung (200) angeordnet wird.

4. Verfahren nach einem der vorhergehenden Ansprüche, **dadurch gekennzeichnet, dass** die Konzentration der Lösung (500) während der Beladung des Ausgangskörpers konstant ist.

5. Verfahren nach einem der vorhergehenden Ansprüche, **dadurch gekennzeichnet, dass** der eine die Verteilung des bioaktiven Oberflächenmaterials (502) innerhalb des Ausgangskörpers durch eine Konvektionsströmung bewirkt wird, wobei eine Strömungsrichtung und - geschwindigkeit durch Einstellen von Luftfeuchtigkeitsunterschieden und/oder Druckunterschieden und/oder Temperaturunterschieden bezüglich verschiedener Oberflächen des Ausgangskörpers gesteuert werden.

6. Verfahren nach einem der vorhergehenden Ansprüche, **dadurch gekennzeichnet, dass** die Konzentration und/oder ein Konzentrationsverlauf der Lösung (500) innerhalb des Ausgangskörpers durch Trocknung und/oder Verdunstung gesteuert wird.

7. Verfahren nach einem der vorhergehenden Ansprüche, **dadurch gekennzeichnet, dass** das bioaktive Oberflächenmaterial (502) kristallisierbar ist, und in einem dritten Schritt, der ein Kristallisationsschritt ist, insbesondere innerhalb des Oberflächenbereichs (102) und/oder im Bereich der ersten Oberfläche (103), kristallisiert wird.

8. Verfahren nach Anspruch 7, **dadurch gekennzeichnet, dass** das bioaktive Oberflächenmaterial (502) form- und/oder kraftschlüssig innerhalb von Poren (107) des Ausgangskörpers, insbesondere innerhalb von Poren (107) des Oberflächenbereichs (102) angeordnet ist.

9. Verfahren nach einem der vorhergehenden Ansprüche, **gekennzeichnet durch** einen Kristallwachstumsschritt, wobei Kristalle des bioaktiven Oberflächenmaterials (502), ausgehend von der Porosität des Oberflächenbereichs (102) zu einer kristallinen Schicht (503) wachsen, welche kristalline Schicht (503) mindestens einen Teil der ersten Oberfläche (103) des Ausgangskörpers bedeckt.

10. Verfahren nach einem der Ansprüche 7 - 9, **gekennzeichnet durch** einen Porenbildungsschritt, wobei die Kristalle und/oder die kristalline Schicht (503) des bioaktiven Materials (502) zur Bildung von Poren und/oder zur Erhöhung der Oberflächenrauigkeit (506) einer Wärmebehandlung unterzogen werden.

11. Verfahren nach einem der vorhergehenden Ansprüche, **gekennzeichnet durch** einen Wärmeausdehnungskoeffizienten-Ausgleichsschritt, wobei der Ausgangskörper mit einer Ausgleichslösung, die Zirkonium und/oder Calcium und/oder Cer aufweist, gespült wird.

12. Verfahren nach einem der vorhergehenden Ansprüche, **gekennzeichnet durch** einen Sinterschritt, wobei mittels des Sinterschritts ein Stoffschluss und/oder ein chemischer Verbund zwischen dem bioaktiven Oberflächenmaterial (502) und dem Trägermaterial erzeugt wird.

13. Verfahren nach einem der vorhergehenden Ansprüche, **gekennzeichnet durch** die nachfolgenden Schritte,
(A) Bereitstellen des porösen Ausgangskörpers
(B) Platzieren der ersten Oberfläche (103) des Ausgangskörpers innerhalb einer abgeschlossenen Umgebung (200) wobei die zweite Oberfläche (104, 106) oder mindestens ein Teil der zweiten Oberfläche (104, 106) des Ausgangskörpers, die eine Beladungsfläche (104, 106), ist außerhalb der abgeschlossenen Umgebung (200) angeordnet ist,
(C)Beladen der Beladungsfläche (104, 106) des Ausgangskörpers mit dem bioaktiven Oberflächenmaterial (502), wobei das bioaktive Oberflächenmaterial (502) in einem Lösungsmittel (500) gelöst ist,
(D) Steuern der Verteilung des bioaktiven Oberflächenmaterials (502), wobei die Lösung (500) innerhalb des Oberflächenbereichs (102) eine höhere Konzentration aufweist als innerhalb des Kernbereichs (101) und mindestens ein Umgebungsparameter, insbesondere die Luftfeuchtigkeit und/oder der Druck und/oder die Temperatur zur Erzeugung eines Umgebungsparametergradienten geregelt wird,
(F) Kristallisation des bioaktiven Oberflächenmaterials (502), wobei die Konzentration der Lösung (500) innerhalb des Oberflächenbereichs (102) durch Verdunstung und/oder Verdampfung und/oder Trocknung erhöht wird,
(G)Ausbilden einer kristallinen Schicht (503) des bioaktiven Oberflächenmaterials (502) durch Kristallwachstum.

## Claims

1. Method for the production of an implant blank (100), in particular of a dental implant blank, from a source body, said implant blank (100) comprising at least one first region, which is a surface region (102), and a second region which is a core region (101), wherein the surface region (102) comprises at least one bioactive surface material (502) and extends from at least one surface (103) going outwards in the direction of the core region (101), and the core region (101) comprises at least one carrier material which can tolerate a mechanical load, **characterized in that** the source body has a porosity for controlling a specifically targeted distribution of the bioactive surface material (502) inside the source body, and in a first step, which is a loading step, is loaded with a solution (500) of the bioactive surface material (502), and in a second step, which is a distribution control step, the distribution of the bioactive surface material (502) inside the source body by means of a convection flow, wherein a flow direction and flow speed are controlled by the specifically targeted production of environmental parameter gradients in relation to different surfaces of the source body in such a way that the solution (500) inside the surface region (102) exhibits a higher concentration than inside the core region (101), wherein the control is applied by the regulation of one or more environmental parameters in a closed environment (200), in particular by regulation of the air humidity and/or the pressure and/or the temperature.

2. Method according to claim 1, **characterized in that** the loading of the source body with the solution (500) of the at least one bioactive surface material (502) takes place over at least one second surface of the source body, which is a loading surface (104, 106), wherein the loading surface (104, 106) is a surface which is different from the first surface (103).

3. Method according to claim 2, **characterized in that** the loading surface (104, 106) for the loading of the source body with the bioactive surface material (502) is arranged outside the closed environment (200).

4. Method according to any one of the preceding claims, **characterized in that** the concentration of the solution (500) is constant during the loading of the source body

5. Method according to any one of the preceding claims, **characterized in that** the distribution of the bioactive surface material (502) inside the source body is put into effect by a convection current, wherein a flow direction and flow speed are controlled by the adjustment of differences in air humidity and/or differences in pressure and/or differences in temperature in respect of different surfaces of the source body.

6. Method according to any one of the preceding claims, **characterized in that** the concentration and/or a concentration profile of the solution (500) inside the source body is controlled by drying and/or evaporation.

7. Method according to any one of the preceding claims, **characterized in that** the bioactive surface material (502) can be crystallised, and in a third step, which is a crystallization step, it is crystallized, in particular inside the surface region (102) and/or in the region of the first surface (103).

8. Method according to claim 7, **characterized in that** the bioactive surface material (502) is arranged in positive and/or non-positive fit inside pores (107) of the source body, in particular inside pores (107) of the surface region (102).

9. Method according to any one of the preceding claims, **characterized by** a crystal growth step, wherein crystals of the bioactive surface material (502), deriving from the porosity of the surface region (102), grow to a crystalline layer (503), said crystalline layer (503) covering at least a part of the first surface (103) of the source body.

10. Method according to any one of claims 7-9, **characterized by** a pore formation layer, wherein the crystals and/or the crystalline layer (503) of the bioactive material (502) are subjected to a heat treatment for the formation of pores and/or for increasing the surface roughness (506).

11. Method according to any one of the preceding claims, **characterized by** a thermal expansion coefficient equalization step, wherein the source body is flushed with an equalization solution, which comprises zirconium and/or calcium and/or cerium.

12. Method according to any one of the preceding claims, **characterized by** a sintering step, wherein, by means of the sintering step, a material connection and/or a chemical bond is produced between the bioactive surface material (502) and the carrier material.

13. Method according to any one of the preceding claims, **characterized by** the following steps:
(A) Provision of the porous source body,
(B) Placement of the first surface (103) of the source body inside a closed environment (200), wherein the second surface (104, 106) or at least a part of the second surface (104, 106) of the source body, which is a loading surface (104, 106), is arranged outside the closed environment (200),
(C) Loading of the loading surface (104, 106) of the source body with the bioactive surface material (502), wherein the bioactive surface material (502) is put into solution with a solvent (500),
(D) Controlling of the distribution of the bioactive surface material (502), wherein the solution (500) inside the surface region (102) exhibits a higher concentration than inside the core region (101), and at least one environmental parameter, in particular the air humidity and/or the pressure and/or the temperature is regulated in order to produce an environmental parameter gradient,
(F) Crystallisation of the bioactive surface material (502), wherein the concentration of the solution (500) inside the surface region (102) is increased by evaporation and/or drying,
(G) Forming of a crystalline layer (503) of the bioactive surface material (502) by crystal growth.

## Revendications

1. Procédé de fabrication d'une ébauche d'implant (100), notamment d'une ébauche d'implant dentaire à partir d'un corps de départ, laquelle ébauche d'implant (100) comprend au moins une première zone qui est une zone de surface (102) et une deuxième zone qui est une zone de noyau (101), la zone de surface (102) présentant au moins un matériau de surface bioactif (502) et s'étendant à partir d'au moins une première surface (103) en direction de la zone de noyau (101), et la zone de noyau (101) présentant au moins un matériau de support pouvant être sollicité mécaniquement, **caractérisé en ce que** le corps de départ présente une porosité pour commander une répartition ciblée du matériau de surface bioactif (502) à l'intérieur du corps de départ et, dans une première étape qui est une étape de chargement, est chargé avec une solution (500) du matériau de surface bioactif (502), et dans une deuxième étape, qui est une étape de commande de répartition, la répartition du matériau de surface bioactif (502) à l'intérieur du corps de départ est effectuée par un écoulement de convection, une direction et une vitesse d'écoulement étant commandées en créant de manière ciblée des gradients de paramètres environnementaux par rapport à différentes surfaces du corps de départ, de telle sorte que la solution (500) présente une concentration plus élevée à l'intérieur de la zone de surface (102) qu'à l'intérieur de la zone de noyau (101), la commande étant effectuée par régulation d'un ou plusieurs paramètres environnementaux dans un environnement fermé (200), notamment par régulation de l'humidité de l'air et/ou de la pression et/ou de la température.

2. Procédé selon la revendication 1, **caractérisé en ce que** le chargement du corps de départ avec la solution (500) de l'au moins un matériau de surface bioactif (502) s'effectue par l'intermédiaire d'au moins une deuxième surface du corps de départ, qui est une surface de chargement (104, 106), la surface de chargement (104, 106) étant une surface différente de la première surface (103).

3. Procédé selon la revendication 2, **caractérisé en ce que** la surface de chargement (104, 106) pour charger le corps de départ avec le matériau de surface bioactif (502) est agencée à l'extérieur de l'environnement fermé (200).

4. Procédé selon l'une quelconque des revendications précédentes, **caractérisé en ce que** la concentration de la solution (500) est constante pendant le chargement du corps de départ.

5. Procédé selon l'une quelconque des revendications précédentes, **caractérisé en ce que** la répartition du matériau de surface bioactif (502) à l'intérieur du corps de départ est effectuée par un écoulement de convection, une direction et une vitesse d'écoulement étant commandées en ajustant les différences d'humidité de l'air et/ou les différences de pression et/ou les différences de température par rapport aux différentes surfaces du corps de départ.

6. Procédé selon l'une quelconque des revendications précédentes, **caractérisé en ce que** la concentration et/ou l'évolution de la concentration de la solution (500) à l'intérieur du corps de départ est commandée par séchage et/ou évaporation.

7. Procédé selon l'une quelconque des revendications précédentes, **caractérisé en ce que** le matériau de surface bioactif (502) est cristallisable, et est cristallisé dans une troisième étape qui est une étape de cristallisation, notamment à l'intérieur de la zone de surface (102) et/ou dans la zone de la première surface (103).

8. Procédé selon la revendication 7, **caractérisé en ce que** le matériau de surface bioactif (502) est agencé à l'intérieur des pores (107) du corps de départ, notamment à l'intérieur des pores (107) de la zone de surface (102), par complémentarité de forme et/ou par adhérence.

9. Procédé selon l'une quelconque des revendications précédentes, **caractérisé par** une étape de croissance cristalline, dans laquelle des cristaux du matériau de surface bioactif (502) croissent à partir de la porosité de la zone de surface (102) en une couche cristalline (503), laquelle couche cristalline (503) recouvre au moins une partie de la première surface (103) du corps de départ.

10. Procédé selon l'une quelconque des revendications 7 à 9, **caractérisé par** une étape de formation de pores, les cristaux et/ou la couche cristalline (503) du matériau bioactif (502) étant soumis à un traitement thermique pour former des pores et/ou augmenter la rugosité de surface (506).

11. Procédé selon l'une quelconque des revendications précédentes, **caractérisé par** une étape de compensation du coefficient de dilatation thermique, le corps de départ étant rincé avec une solution de compensation présentant du zirconium et/ou du calcium et/ou du cérium.

12. Procédé selon l'une quelconque des revendications précédentes, **caractérisé par** une étape de frittage ; au moyen de l'étape de frittage, une liaison de matière et/ou une liaison chimique étant créée entre le matériau de surface bioactif (502) et le matériau de support.

13. Procédé selon l'une quelconque des revendications précédentes, **caractérisé par** les étapes suivantes,
(A) la fourniture du corps de départ poreux,
(B) le placement de la première surface (103) du corps de départ à l'intérieur d'un environnement fermé (200), la deuxième surface (104, 106) ou au moins une partie de la deuxième surface (104, 106) du corps de départ, qui est une surface de chargement (104, 106), étant agencée à l'extérieur de l'environnement fermé (200),
(C) le chargement de la surface de chargement (104, 106) du corps de départ avec le matériau de surface bioactif (502), le matériau de surface bioactif (502) étant dissous dans un solvant (500),
(D) la commande de la répartition du matériau de surface bioactif (502), la solution (500) ayant une concentration plus élevée à l'intérieur de la zone de surface (102) qu'à l'intérieur de la zone de noyau (101), et au moins un paramètre environnemental, notamment l'humidité de l'air et/ou la pression et/ou la température étant régulé pour créer un gradient de paramètre environnemental,
(F) la cristallisation du matériau de surface bioactif (502), la concentration de la solution (500) à l'intérieur de la zone de surface (102) étant augmentée par évaporation et/ou vaporisation et/ou séchage,
(G) la formation d'une couche cristalline (503) du matériau de surface bioactif (502) par croissance cristalline.
